(19) Europäisches Patentamt
European Patent Office
Office européen des brevets

(11) **EP 2 916 733 B1**

(12) **EUROPÄISCHE PATENTSCHRIFT**

(45) Veröffentlichungstag und Bekanntmachung des Hinweises auf die Patenterteilung:
**26.10.2016 Patentblatt 2016/43**

(51) Int Cl.:
*A61B 5/107* (2006.01)     *G01B 17/00* (2006.01)
*G01B 5/06* (2006.01)       *A61B 5/00* (2006.01)

(21) Anmeldenummer: **13750048.4**

(22) Anmeldetag: **14.08.2013**

(86) Internationale Anmeldenummer:
**PCT/EP2013/067022**

(87) Internationale Veröffentlichungsnummer:
**WO 2014/072089 (15.05.2014 Gazette 2014/20)**

(54) **LÄNGENMESSGERÄT**

LENGTH MEASURING DEVICE

APPAREIL DE MESURE DE LONGUEUR

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **09.11.2012 DE 102012220468**

(43) Veröffentlichungstag der Anmeldung:
**16.09.2015 Patentblatt 2015/38**

(73) Patentinhaber: **seca ag**
**4153 Reinach BL 1 (CH)**

(72) Erfinder:
• **GROßMANN, Jan-Erik**
**22527 Hamburg (DE)**

• **JENSEN, Björn**
**22043 Hamburg (DE)**
• **LAWITZKE, Peter**
**23847 Rethwisch (DE)**

(74) Vertreter: **Uexküll & Stolberg**
**Partnerschaft von**
**Patent- und Rechtsanwälten mbB**
**Beselerstraße 4**
**22607 Hamburg (DE)**

(56) Entgegenhaltungen:
**EP-A1- 0 940 658        WO-A1-98/17974**
**AT-B- 397 430           AT-B- 401 109**
**DE-A1-102010 001 886    JP-A- 2004 061 362**
**JP-A- 2009 042 142**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die vorliegende Erfindung betrifft ein Längenmessgerät mit einem Messschieber, einer linearen Führung in Form eines Hohlprofils, an dem der Messschieber außen verschiebbar gelagert ist, um ihn in Anlage an ein bezüglich seiner Länge zu messendes Objekt bringen zu können, einem inneren Schieber, der im Inneren des Hohlprofils verschiebbar gelagert ist, einer Magnetanordnung, die Messschieber und inneren Schieber magnetisch koppelt, so dass der innere Schieber jeder Bewegung des Messschiebers entlang des Hohlprofils folgt, einer Messeinrichtung zur Messung der Position des inneren Schiebers entlang des Hohlprofils und einer im Außenraum des Hohlprofils sichtbaren Anzeige der durch die Messeinrichtung ermittelten Länge nach Maßgabe der gemessen Position des inneren Schiebers.

Das Hohlprofil ist an wenigstens einem Ende mit einer Endwand versehen. Ferner deckt der innere Schieber den Querschnitt des Hohlprofils im Wesentlichen ab, so dass im Innenraum des Hohlprofils zwischen der Endwand und dem inneren Schieber ein geschlossener Hohlraum gebildet ist, der als akustischer Resonator dienen kann.

Die Erfindung ist insbesondere auf Längenmessgeräte zur Messung der Körpergröße von Personen gerichtet. Solche Längenmessgeräte werden auch als Stadiometer bezeichnet. Ein typisches derartiges Längenmessgerät weist einen Messstab auf, der als vertikale lineare Führung für einen Messschieber (Kopfschieber) ausgebildet ist. Der Messstab ist vertikal ausgerichtet und an einer Wand montiert oder an einer Plattform befestigt. Zum Messen der Körpergröße tritt die Person vor den Messstab, wonach der Kopfschieber an dem Messstab heruntergeschoben wird, bis er in Auflage auf den Kopf der zu messenden Person kommt. An dem Messstab ist eine Messskala mit Skaleneinteilungsstrichen vorgesehen. In dem Messschieber ist wiederum eine Leseeinheit vorhanden, die die Skaleneinteilungsstriche beim Verschieben des Messschiebers an dem Messstab registriert und somit die inkrementelle Änderung der Position des Messschiebers erfasst. In den Skaleneinteilungsstrichen kann auch die absolute Höhe eines Skaleneinteilungsstrichs kodiert sein, so dass mit der Leseeinheit die Höhe des Kopfschiebers bestimmt werden kann, die dann auf einer Anzeige an dem Messschieber angezeigt wird.

Ein anderer Typ eines Längenmessgerätes für die Körpergröße weist ein Winkelstück auf, das von einer die Messung der Körperlänge durchführenden Person gehalten wird. Ein Schenkel des Winkelstücks wird in Auflage auf den Kopf der zu messenden Person gehalten. Von diesem Schenkel in Auflage auf dem Kopf steht ein zweiter Schenkel senkrecht ab, wobei das Winkelstück so gehalten wird, dass der zweite Schenkel vertikal auf den Boden gerichtet verläuft. In dem zweiten Schenkel ist eine Abstandsmesseinrichtung mit einem Ultraschallwandler vorgesehen, die aus der Laufzeit eines ausgesendeten Ultraschallsignals, das am Boden reflektiert wird und zum Ultraschallwandler zurückläuft, die Höhe des in Auflage auf dem Kopf der zu messenden Person befindlichen ersten Schenkels über dem Boden und damit die Körpergröße bestimmt und anzeigt. Ein Nachteil dieses Typs von Längenmessgerät besteht darin, dass Messungenauigkeiten dadurch auftreten können, dass die die Messung durchführende Person das Winkelstück nicht exakt so ausgerichtet hält, dass der zweite Schenkel genau vertikal auf den Boden gerichtet ist. Weiterhin ist nachteilig, dass wechselnde Umgebungsbedingungen (z.B. Staub oder anderer Verunreinigungen in der Luft) oder auf dem Boden liegende Gegenstände die Messung verfälschen können.

Ein weiterer Typ eines Längenmessgerätes weist eine Plattform, auf die die zu messende Person tritt, und einen vertikal oberhalb der zu messenden Personen fest aufgehängten horizontalen Träger auf. An dem Träger ist eine Abstandsmesseinrichtung auf Basis von Ultraschallwellenlaufzeit angebracht, die auf den Kopf der auf der Plattform stehenden, zu messenden Person gerichtet ist. Die zu messende Person trägt eine Kappe, um eine wohldefinierte Reflexion der Ultraschallwellen von der Oberseite des Kopfes zu gewährleisten. Aus dem Abstand der Oberseite des Kopfes der Person zu der vertikal oberhalb der zu messenden Person fest installierten Abstandsmesseinrichtung lässt sich aus der Differenz der Aufhängungshöhe der Abstandsmesseinrichtung und dem gemessenen Abstand zur Oberseite des Kopfes die Körperlänge der zu messenden Person ableiten. Auch dieses Längenmessgerät ist fehleranfällig, da die Messung durch wechselnde Umgebungsbedingungen und störende Einflüsse in der offenen Messstrecke zwischen dem Ultraschallwandler und der Oberseite des Kopfes der zu messenden Person verfälscht werden kann.

Aus WO 98/17974 A1 ist ein Längenmessgerät mit den Merkmalen des Oberbegriffs von Anspruch 1 bekannt. Dieses Längenmessgerät ist nicht als Längenmessgerät für Personen ausgebildet. Vielmehr soll die Position eines Messschiebers verfolgt werden. Das Längemessgerät hat eine lineare Führung in Form eines hohlen Gehäuses, an dem der Messschieber außen verschiebbar gelagert ist. Im Inneren des Gehäuses ist ein innerer Schieber darin verschiebbar gelagert. Eine Magnetanordnung koppelt den Messschieber und den inneren Schieber magnetisch, so dass der innere Schieber jeder Bewegung des Messschiebers entlang der Führung folgt. Der innere Schieber ist in Schleifkontakt mit einem linearen Potentiometer, um ein Spannungssignal bereitzustellen, das proportional zu einer Position des inneren Schiebers ist. Aus dem Potentiometersignal wird die Position des inneren Schiebers und damit die des damit gekoppelten Messschiebers entlang der linearen Führung abgeleitet. Durch die Messung der Position des inneren Schiebers im Inneren des Gehäuses sind zwar gewissen Störfaktoren wie Rauch oder Staub, die nicht in das Innere des Gehäuses eindringen, in ihrem Einfluss auf die Messgenauigkeit reduziert. Das Potentiometer weist Schleifkontakte auf, die Abrieb erzeugen und dadurch mit der Zeit verschleißen. Um dem entgegen zu wirken, müssen hochwertige Materialien eingesetzt werden, was natürlich die Herstellungskosten erhöht. Aber auch unter diesen Bedingungen kann der Verschleiß zu einer Verschlechterung der Messgenauigkeit führen.

Es ist Aufgabe der vorliegenden Erfindung, ein Längenmessgerät so auszubilden, dass es unabhängig von veränderlichen Umgebungsbedingungen eine hohe Messgenauigkeit bietet und keine verschleißbedingte Beeinträchtigung der Messgenauigkeit zeigt. Zur Lösung dieser Ausgabe dient ein Längenmessgerät mit den Merkmalen des Patentanspruchs 1. Vorteilhafte Ausführungsformen der Erfindung sind in den Unteransprüchen aufgeführt.

Erfindungsgemäß weist die Messeinrichtung einen Lautsprecher und ein Mikrophon im Inneren des akustischen Resonators zwischen der Endwand und dem inneren Schieber auf. Eine mit dem Mikrophon und dem Lautsprecher verbundene Steuer- und Auswerteeinheit ist dazu eingerichtet, den Lautsprecher zur Abgabe eines akustischen Impulses zu veranlassen und dann mit dem Mikrophon die Impulsantwort des akustischen Resonators aufzunehmen. Die Steuer- und Auswerteeinheit ist weiter dazu eingerichtet, aus der Impulsantwort des akustischen Resonators dessen Grundfrequenz zu bestimmen und aus der Grundfrequenz die Länge des Resonators und damit die Position des inneren Schiebers entlang des Hohlprofils zu bestimmen. Unter Grundfrequenz wird hier die niedrigste Eigenfrequenz verstanden, entsprechend einer stehenden Welle, deren Wellelänge doppelt so lang wie der akustische Resonator ist.

Der innere Schieber deckt den Querschnitt im Wesentlichen ab, um auftreffende Schallwellen effektiv zu reflektieren, kann aber auch eine kleine Öffnung haben, z.B. um bei seiner Bewegung Luft durchzulassen.

In einer vorteilhaften Ausführungsform ist die Steuer- und Auswerteeinheit dazu eingerichtet, die aufgenommene Impulsantwort des akustischen Resonators durch eine Fourier-Transformation in ein Frequenzspektrum zu transformieren und in dem Frequenzspektrum die Grundfrequenz zu bestimmten. Vorzugsweise wird die Fourier-Transformation in Form einer diskreten Fourier-Transformation (DFT) oder einer schnellen Fourier-Transformation (FFT) durchgeführt.

In einer bevorzugten Ausführungsform ist die Steuer- und Auswerteeinheit dazu eingerichtet, bei der Bestimmung der Grundfrequenz in dem Frequenzspektrum den Abstand von zwei aufeinanderfolgenden Maxima zu bestimmen. Der Abstand kann dazu verwendet werden, um zu jedem Maximum im Frequenzspektrum festzustellen, um die wievielte Oberwelle es sich handelt. Mit dieser Information kann dann aus jedem Maximum eine Grundfrequenz bestimmt werden, und diese dann zu einer Grundfrequenz gemittelt werden. Es können auch mehrere Abstände aufeinanderfolgender Maxima gemittelt werden, um die Grundfrequenz zu bestimmen.

Vorzugsweise ist die Steuer- und Auswerteeinheit dazu eingerichtet, die Bandbreite des über das Mikrophon erzeugten akustischen Impulses so zu wählen, dass die kleinste enthaltene Wellenlänge größer als der doppelte Durchmesser des Hohlprofils ist.

Dadurch wird gewährleistet, dass sich der Schall in dem Hohlprofil als ebene Welle ausbreitet. Bei kürzeren Wellenlängen könnte der Schall auch zwischen den Wänden des Hohlprofils reflektiert werden und damit auch weitere Moden anregen, deren Schallgeschwindigkeit in Längsrichtung des Hohlprofils kleiner als die Schallgeschwindigkeit einer ebenen Welle sind; diese würden das Messverfahren zwar nicht verfälschend beeinflussen, da sie bei der Fourier-Transformation ja getrennt würden und dann nur nicht zur Auswertung herangezogen werden sollten. Die Schallenergie solcher eventueller weiteren Moden wäre dann aber nicht für die Messung nutzbar und somit sinnlos erzeugt, weswegen die Anregung einer reinen ebenen Welle bevorzugt ist.

In einer bevorzugten Ausführungsform ist das Hohlprofil auch am anderen Ende mit einer zweiten Endwand versehen, um im Innenraum des Hohlprofils zwischen der zweiten Endwand und dem inneren Schieber einen zweiten akustischen Resonator zu bilden. In diesem zweiten akustischen Resonator sind ein zweiter Lautsprecher und ein zweites Mikrophon angeordnet. Die Steuer- und Auswerteeinheit ist weiter dazu eingerichtet, den zweiten Lautsprecher zur Abgabe eines akustischen Impulses zu veranlassen, mit dem zweiten Mikrophon die Impulsantwort des zweiten akustische Resonators aufzunehmen, daraus die Grundfrequenz des zweiten akustischen Resonators zu bestimmen und aus der Grundfrequenz die Länge des zweien Resonators und damit die Position des inneren Schiebers entlang des Hohlprofils zu bestimmen. Bei einer derartigen Längenmessvorrichtung kann dann die Steuer- und Auswerteeinheit vorteilhaft weiter dazu eingerichtet sein, mithilfe der bestimmten Längen des akustischen Resonators, des zweiten akustischen Resonators, der bekannten Länge des Hohlprofils zwischen der Endwand und der zweiten Endwand und der bekannten axialen Länge des inneren Schiebers eine Kalibration der Längenbestimmungen durchführen zu können.

In einer bevorzugten Ausführungsform weist die Magnetanordnung wenigstens einen Permanentmagneten an dem Messschieber und einen Permanentmagneten an dem inneren Schieber auf, die so angeordnet sind, dass entgegengesetzte Pole der beiden Permanentmagnete aufeinander zu weisend zueinander ausgerichtet sind. Vorzugsweise sind jeweils vier Permanentmagnete an dem inneren Schieber und an dem Messschieber so zueinander angeordnet, dass jeweils ein Paar eines Permanentmagneten an dem Messschieber und an dem inneren Schieber mit entgegengesetzten Polen aufeinander zu weisend zueinander ausgerichtet ist. Alternativ weist die Magnetanordnung nur einen Permanentmagneten an einem von Messschieber und innerem Schieber auf, wobei dann die andere Komponente von Messschieber und innerem Schieber ferro- oder paramagnetisches Material enthält, so dass Messschieber und innerer Schieber magnetisch gekoppelt sind.

Die äußeren Abmessungen des inneren Schiebers sind vorzugsweise an die inneren Abmessungen des Hohlprofils angepasst, so dass der innere Schieber möglichst spielfrei, aber gleitfähig in dem Hohlprofil sitzt. Entsprechend sind die inneren Abmessungen des Messschiebers so an die äußeren Abmessungen des Hohlprofils angepasst, dass der Messschieber mit möglichst geringem Spiel, aber gleitfähig außen auf dem Hohlprofil gelagert ist.

Die Erfindung wird im Folgenden anhand eines Ausführungsbeispiels im Zusammenhang mit den Zeichnungen beschrieben, in denen:

Fig. 1 eine schematische Ansicht eines Längenmessgerätes zeigt,

Fig. 2 eine Explosionsdarstellung von Teilen des Längenmessgerätes im Bereich des Messschiebers im Längsschnitt zeigt,

Fig. 3 eine Querschnittsansicht des Längenmessgerätes im Bereich von innerem Schieber und Messschieber zeigt, wobei die Schieberkomponenten selbst nicht dargestellt sind, sondern lediglich die in ihnen angeordneten Permanentmagnete,

Fig. 4 eine schematische Ansicht des Hohlprofils einer Längenmessvorrichtung im Längsschnitt zeigt,

Fig. 5 den zeitlichen Amplitudenverlauf eines den Lautsprecher zur Abgabe eines akustischen Impulses anregenden Signals als Funktion der Zeit zeigt,

Fig. 6 das aufgenommene Mikrophonsignal der Impulsantwort des akustischen Resonators in dem Hohlprofil zeigt,

Fig. 7 die Impulsantwort des akustischen Resonators aus Fig. 6 transformiert in ein Frequenzspektrum zeigt und

Fig. 8 eine schematische Ansicht des Hohlprofils im Längsschnitt einer weiteren Ausführungsform für eine Längenmessvorrichtung zeigt.

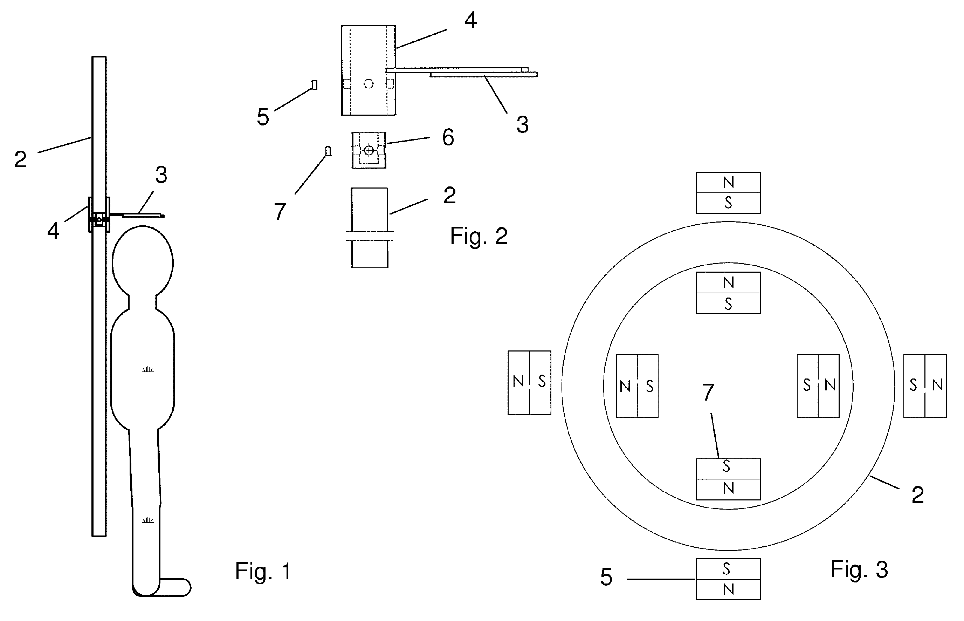

Fig. 1 zeigt eine Seitenansicht eines Längenmessgerätes, das zum Beispiel an einer Wand befestigt sein kann. Das Längenmessgerät weist ein Hohlprofil 2 als lineare Führung auf, auf der außen verschiebbar ein Messschieber 4 gelagert ist, der eine Kopfplatte 3 trägt. Der Messschieber 4 wird soweit abgesenkt, bis die Kopfplatte 3 oben auf dem Kopf der zu messenden Person aufliegt.

In dem Hohlprofil 2, das in diesem Ausführungsbeispiel im Querschnitt kreisringförmig ist, ist ein innerer Schieber 6 verschiebbar gelagert (siehe Fig.2). Der innere Schieber 6 ist im Querschnitt scheibenförmig oder, wie hier dargestellt, mit einer geschlossenen unteren Endwand versehen, so dass der innere Schieber 6 den Querschnitt des Hohlprofils 2 abdeckt. Die äußeren Abmessungen des inneren Schiebers 6 sind an die inneren Abmessungen des Hohlprofils 2 angepasst, so dass der innere Schieber 6 mit möglichst geringem Spiel, aber gleitfähig im Innenraum des Hohlprofils 2 sitzt. Entsprechend sind die inneren Abmessungen des Messschiebers 4 an die äußeren Abmessungen des Hohlprofils 2 angepasst, so dass der Messschieber 4 mit möglichst geringem Spiel, aber gleitfähig auf dem äußeren Umfang des Hohlprofils 2 gelagert ist.

Fig. 3 zeigt einen Querschnitt durch das Hohlprofil 2 im Bereich des Messschiebers und des inneren Schiebers, wobei die Messschieber- und die innere Schieberkomponente selbst nicht dargestellt sind, sondern nur die Magnetanordnung aus mehreren Permanentmagneten, die in dem inneren Schieber und dem Messschieber eingebracht sind. In dem inneren Schieber 6 sind um den Umfang herum verteilt vier Permanentmagnete 7 eingebracht, die in einem Abstand von 90° zueinander um den Umfang verteilt sind. Entsprechend sind in dem Messschieber 4 ebenfalls vier Permanentmagnete 5 eingebracht, die in entsprechenden Abständen von 90° um den äußeren Messschieber verteilt angeordnet sind. Dabei ist die Anordnung der Permanentmagnete so, dass einander gegenüberliegende Permanentmagnete 5 und 7 des Messschiebers 4 und des inneren Schiebers 6 mit entgegen gesetzten Polen aufeinander zu ausgerichtet sind. In dem dargestellten Ausführungsbeispiel wird dies dadurch erreicht, dass die Permanentmagnete 7 des inneren Schiebers mit einem magnetischen Pol, in diesem Beispiel mit dem Nordpol, nach außen ausgerichtet sind, während die Permanentmagnete 5 ebenfalls mit diesem Magnetpol, hier dem Nordpol, nach außen gerichtet angeordnet sind, so dass sich jeweils ein Paar von Permanentmagneten 5 und 7 mit entgegen gesetzten Polen aufeinander zu gerichtet gegenüberliegt. Auf diese Weise sind der Messschieber 4 und der innere Schieber 6 magnetisch miteinander gekoppelt. Dadurch folgt der innere Schieber 6 jeder Bewegung des Messschiebers 4 entlang des Hohlprofils 2. In Fig. 2 ist jeweils nur einer der Permanentmagnete 5 und 7 gezeigt.

Grundsätzlich können natürlich auch mehr oder weniger als vier Permanentmagnete pro Schieberkomponente vorgesehen sein, z.B. nur jeweils ein Permanentmagnet in dem Messschieber 4 und dem inneren Schieber 6. Es ist sogar möglich, das überhaupt nur ein Magnet entweder in dem inneren Schieber 6 oder in dem Messschieber 4 vorgesehen ist und die andere Schieberkomponente ohne eigenen Magneten ferro- oder paramagnetisches Material enthält, so dass eine magnetische Anziehung zwischen dem inneren Schieber 6 und dem Messschieber 4 bewirkt wird. Der oder die Magnete der Magnetanordnung sind vorzugsweise Permanentmagnete, grundsätzlich sind aber auch Elektromagnete einsetzbar.

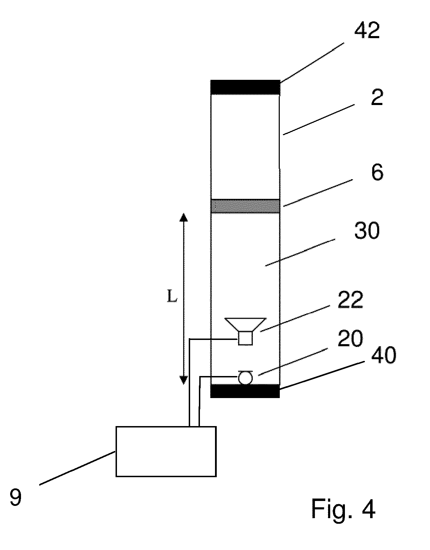

Fig. 4 zeigt eine schematische Längsschnittansicht eines Hohlprofils 2 einer Längenmessvorrichtung. In diesem Ausführungsbeispiel ist der innere Schieber 6 einfach scheibenförmig ausgebildet und deckt den Querschnitt des Hohlprofils 2 ab. Das Hohlprofil 2 ist am unteren Ende mit einer geschlossenen Endwand 40 versehen, so dass zwischen der Endwand 40 und dem inneren Schieber 6 ein geschlossener Raum in dem Hohlprofil gebildet ist, der als akustischer Resonator betrachtet werden kann. Zu der Messeinrichtung zur Bestimmung der Position des inneren Schiebers 6 entlang der Längsrichtung des Hohlprofils 2 gehören ein Lautsprecher 22 und ein Mikrophon 20, die im Inneren des akustischen Resonators 30 angeordnet sind. Der Lautsprecher 22 und das Mikrophon 20 sind mit einer Steuer- und Auswerteeinheit 9 verbunden, die hier im Außenraum des Hohlprofils angeordnet gezeigt ist, die aber auch im Innenraum des Hohlprofils untergebracht sein kann. Die Steuer- und Auswerteeinheit 9 ist eine programmierbare Datenverarbeitungseinrichtung, die dazu eingerichtet ist, den Lautsprecher 22 zur Abgabe eines akustischen Impulses anzuregen und die resultierende Impulsantwort des akustischen Resonators 30 durch Aufnahme des Signals des Mikrophons 20 aufzunehmen. Die Steuer- und Auswerteeinheit 9 ist weiter dazu eingerichtet, aus der akustischen Impulsantwort die Grundfrequenz $f_0$ des Resonators zu bestimmen. Diese Grundfrequenz $f_0$ besitzt eine Wellenlänge $\lambda_0$, die der zweifachen Länge des akustischen Resonators entspricht (die einfachste stehende Welle in dem akustischen Resonator 30 ist eine Halbwelle mit jeweils einem Geschwindigkeitsknoten an der Endwand 40 und an der reflektierenden Wand des inneren Schiebers 6). Mit Kenntnis der Schallgeschwindigkeit c und der Grundfrequenz $f_0$ lässt sich somit die Länge des Resonators bestimmen:

$$L = \frac{c}{2 \cdot f_0}$$

Bei einer breitbandigen Anregung durch einen akustischen Impuls werden im akustischen Resonator 30 neben der Grundfrequenz auch höhere Moden angeregt, deren Wellenlängen ganzzahlige Vielfache von $\lambda_0/2$ sind. Diese Moden können, wie später gezeigt wird, für das Messverfahren zusätzlich von Nutzen sein.

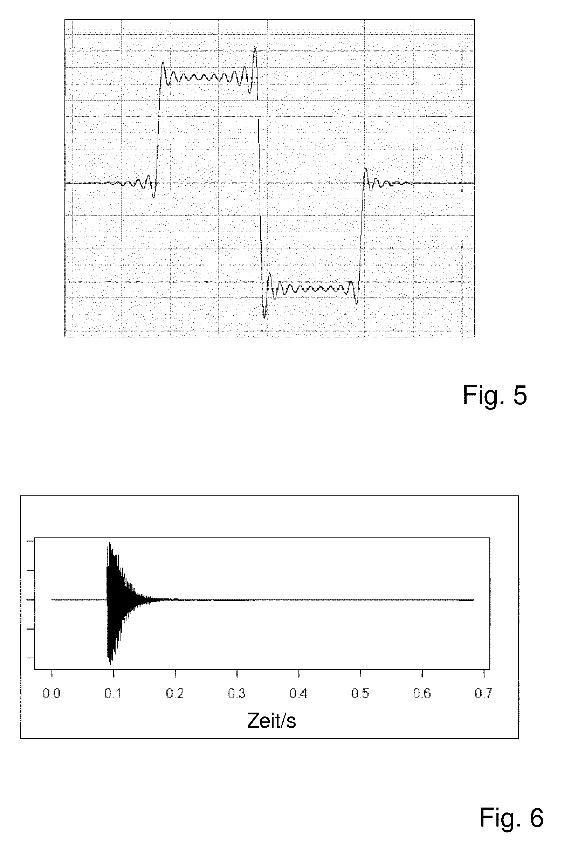

Fig. 5 zeigt die Zeitabhängigkeit eines von der Steuer- und Auswerteeinheit 9 erzeugten Anregungssignals für den Lautsprecher 22 zur Abgabe eines akustischen Impulses. Dieses Signal besteht vorzugsweise aus einer positiven und negativen Halbwelle mit im Wesentlichen Rechteckform. Das Anregungssignal hat keine ideale Rechteckform (die im Prinzip ein nach oben unbegrenztes Frequenzspektrum hätte), da die Bandbreite des Impulses begrenzt wird. Die Bandbreite des Impulses sollte so gewählt werden, dass die kleinste enthaltene Wellenlänge größer als der doppelte Durchmesser des Hohlprofils ist. Damit wird, wie oben bereits erwähnt, gewährleistet, dass sich der Schall im Hohlprofil als ebene Welle ausbreitet. Die zeitliche Länge einer Halbwelle des Anregungssignals sollte größer sein als die Periodendauer des Signals mit der niedrigsten Frequenz, das ausgewertet werden soll.

Fig. 6 zeigt den zeitlichen Verlauf der mit dem Mikrophon 20 aufgenommenen Impulsantwort des akustischen Resonators. Um daraus die Grundfrequenz $f_0$ des akustischen Resonators zu bestimmen, ist es zweckmäßig, die akustische Impulsantwort aus Fig. 6 in ein Frequenzspektrum zu transformieren, um die Grundfrequenz $f_0$ in dem Frequenzspektrum zu ermitteln. Die Transformation in ein Frequenzspektrum erfolgt durch eine Fourier-Transformation. Mögliche Verfahren der digitalen Signalverarbeitung zur Fourier-Transformation sind die beispielsweise diskrete Fourier-Transformation (DFT) oder die schnelle Fourier-Transformation (FFT), zu deren Ausführung die Steuer- und Auswerteeinheit 9 eingerichtet sein kann.

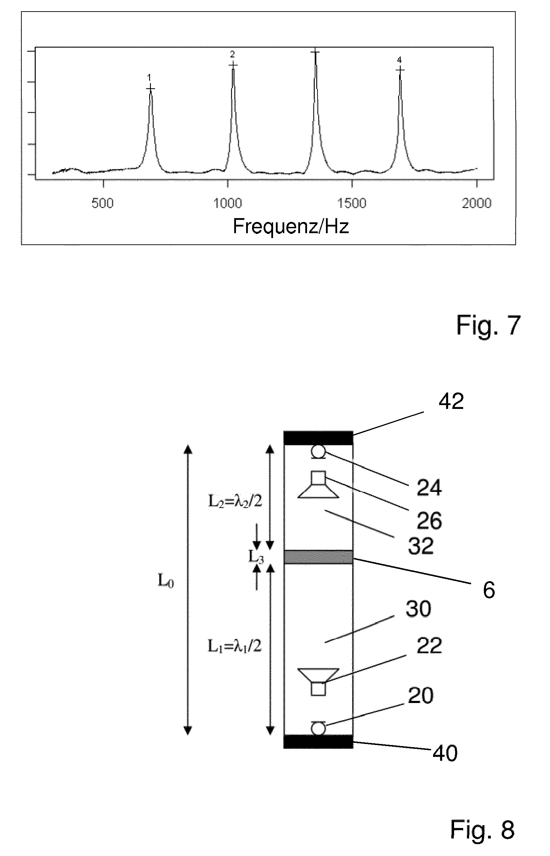

Fig. 7 zeigt nun einen Ausschnitt des Frequenzspektrums der Impulsantwort. Darin sind lokale Maxima, die durch die angeregten Eigenmoden entstehen, klar zu erkennen. Diese treten bei ganzzahligen Vielfachen der Grundfrequenz $f_0$ auf. Die Grundfrequenz kann somit auch über die Abstände benachbarter Moden im Frequenzspektrum ermittelt werden. Diese Verfahrensweise hat den Vorteil, dass die untere Grenzfrequenz des verwendeten Lautsprechers größer gewählt werden kann als die eigentliche zu ermittelnde Grundfrequenz $f_0$ des akustischen Resonators. Die untere Grenzfrequenz eines Lautsprechers wird unter anderem durch die Größe seiner Membran bestimmt. Je größer diese ist, umso tiefer kann die Grenzfrequenz liegen. Da der Lautsprecher im Inneren des Hohlprofils platziert ist, wird ein möglichst kleiner Membrandurchmesser angestrebt. Dadurch kann es geschehen, dass die einfachste stehende Welle in dem akustischen Resonator mit der vorliegenden Verfahrensweise nicht gut beobachtbar ist, da der Lautsprecher die dafür erforderliche große Wellenlänge nicht ausreichend erzeugen kann. Dieser Effekt ist auch in Fig. 7 zu erkennen, aus der sich ergibt, dass die Grundfrequenz etwas mehr als 300 Hz beträgt (Abstände der aufeinanderfolgenden Maxima). Bei etwas mehr als 300 Hz ist in dem in Fig. 7 gezeigten Frequenzspektrum jedoch aus dem angegebenen Grund bei der Grundfrequenz von etwas mehr als 300 Hz kein nutzbares Maximum erkennbar.

Neben der exakten Ermittlung der Grundfrequenz $f_0$ muss für die Steuer- und Auswerteeinheit die Schallgeschwindigkeit gut bekannt sein. Da die Schallgeschwindigkeit eine Temperaturabhängigkeit besitzt, empfiehlt es sich, die Temperatur $\vartheta$ innerhalb des Resonators zu messen und damit die aktuelle Schallgeschwindigkeit zu berechnen.

Dies kann beispielsweise mit der Gleichung

$$c = 331{,}5\frac{m}{s} \cdot \sqrt{1 + \frac{\vartheta}{273{,}15°C}}$$

erfolgen.

In Fig. 8 ist eine weitere Ausführungsform dargestellt, mit der die Genauigkeit der Messung der Messeinrichtung verbessert werden kann. In Fig. 8 ist wie in Fig. 4 eine schematische Ansicht des Hohlprofils im Längsschnitt der Längenmessvorrichtung gezeigt. Wie in der Ausführungsform nach Fig. 6 ist in dem Hohlprofil 2 ein akustischer Resonator 30 ausgebildet, in dem sich ein Lautsprecher 22 und ein Mikrophon 20 befinden (die mit diesen verbundene Steuer- und Auswerteeinheit ist hier zur Vereinfachung der Darstellung fortgelassen). Darüber hinaus ist in dem Hohlprofil zwischen der gegenüberliegenden zweiten Endwand 42 des Hohlprofils und dem inneren Schieber 6 ein zweiter akustischer Resonator 32 ausgebildet. Darin befinden sich ein zweiter Lautsprecher 26 und ein zweites Mikrophon 24, die ebenfalls mit der Steuer- und Auswerteeinheit verbunden sind. Durch diese Anordnung kann die Längenmessung kalibriert werden, da die Länge des Hohlprofils zwischen den Endwänden 40 und 42 und die axiale Länge des inneren Schiebers 6 bekannt sind. Unter der Voraussetzung, dass die Schallgeschwindigkeit in dem ersten akustischen Resonator 30 und in dem zweiten akustischen Resonator 32 gleich ist (was voraussetzt, dass die Temperatur in den beiden akustischen Resonatoren gleich ist), kann die Schallgeschwindigkeit durch folgende Gleichungen bestimmt werden:

$$L_0 = L_1 + L_2 + L_3$$

$$L_0 - L_3 = L_1 + L_2 = \frac{c}{2 \cdot f_{01}} + \frac{c}{2 \cdot f_{02}} = \frac{c}{2}\left(\frac{1}{f_{01}} + \frac{1}{f_{02}}\right)$$

$$c = \frac{2 \cdot (L_0 - L_3)}{\left(\dfrac{1}{f_{01}} + \dfrac{1}{f_{02}}\right)} = 2 \cdot (L_0 - L_3) \cdot \frac{(f_{01} \cdot f_{02})}{f_{01} + f_{02}}$$

mit

$L_0$     Länge des Hohlprofils zwischen den Endwänden 40, 42
$L_1$     Länge des akustischen Resonators 30
$L_2$     Länge des oberen, zweiten Resonators 32
$L_2$     axiale Länge des inneren Schiebers 6 in Längsrichtung des Hohlprofils
$f_{01}$     Grundfrequenz des Resonators 30
$f_{02}$     Grundfrequenz des zweiten Resonators 32

Die so bestimmte Schallgeschwindigkeit kann dann verwendet werden, um zum Beispiel die Länge des einen akustischen Resonators 30 zu bestimmen:

$$L_1 = \frac{c}{2 \cdot f_{01}}$$

Bezugszeichenliste

2     Hohlprofil

| | |
|---|---|
| 3 | Kopfplatte |
| 4 | Messschieber |
| 5 | Permanentmagnete des Messschiebers |
| 6 | Innerer Schieber |
| 7 | Permanentmagnete des inneren Schiebers |
| 9 | Steuer- und Auswerteinheit |
| 20 | Mikrophon |
| 22 | Lautsprecher |
| 24 | zweites Mikrophon |
| 26 | zweiter Lautsprecher |
| 30 | Akustischer Resonator |
| 32 | Zweiter akustischer Resonator |
| 40 | Endwand |
| 42 | Zweite Endwand |

**Patentansprüche**

1. Längenmessgerät mit einem Messschieber (4), einer linearen Führung in Form eines Hohlprofils (2), an dem der Messschieber (4) außen verschiebbar gelagert ist, um ihn in Anlage an ein bezüglich seiner Länge zu messendes Objekt bringen zu können, einem inneren Schieber (6), der im Inneren des Hohlprofils (2) verschiebbar gelagert ist, einer Magnetanordnung (5, 7), die Messschieber (4) und inneren Schieber (6) magnetisch koppelt, so dass der innere Schieber (6) jeder Bewegung des Messschiebers (4) entlang des Hohlprofils (2) folgt, einer Messeinrichtung zur Messung der Position des inneren Schiebers (6) entlang des Hohlprofils (2) und einer im Außenraum des Hohlprofils sichtbaren Anzeige der durch die Messeinrichtung ermittelten Länge nach Maßgabe der gemessenen Position des inneren Schiebers (6), wobei das Hohlprofil (2) an wenigstens einem Ende mit einer Endwand (40) versehen ist und der innere Schieber (6) den Querschnitt des Hohlprofils (2) im Wesentlichen abdeckt, um im Innenraum des Hohlprofils (2) zwischen Endwand (40) und dem inneren Schieber (6) einen akustischen Resonator (30) zu bilden, **dadurch gekennzeichnet, dass** die Messeinrichtung einen Lautsprecher (22) und ein Mikrophon (20) im Inneren des akustischen Resonators (30) sowie eine damit verbundene Steuer- und Auswerteeinheit (9) aufweist, die dazu eingerichtet ist, den Lautsprecher (22) zur Abgabe eines akustischen Impulses zu veranlassen, mit dem Mikrophon (20) die Impulsantwort des akustischen Resonators (30) aufzunehmen, daraus die Grundfrequenz ($f_0$) des akustischen Resonators (30) zu bestimmen und aus der Grundfrequenz die Länge des Resonators (30) und damit die Position des inneren Schiebers (6) entlang des Hohlprofils (2) zu bestimmen.

2. Längenmessgerät nach Anspruch 1, **dadurch gekennzeichnet, dass** die Steuer- und Auswerteeinheit (9) dazu eingerichtet ist, die gemessene Impulsantwort des akustischen Resonators (30) durch eine Fourier-Transformation in ein Frequenzspektrum zu transformieren und in dem Frequenzspektrum die Grundfrequenz zu bestimmen.

3. Längenmessgerät nach Anspruch 2, **dadurch gekennzeichnet, dass** die Steuer- und Auswerteeinheit (9) dazu eingerichtet ist, zur Fourier-Transformation eine diskrete Fourier-Transformation (DFT) oder eine schnelle Fourier-Transformation (FFT) durchzuführen.

4. Längenmessgerät nach Anspruch 2 oder 3, **dadurch gekennzeichnet, dass** die Steuer- und Auswerteeinheit (9) dazu eingerichtet ist, im Frequenzspektrum des akustischen Resonators (30) den Abstand zweier aufeinanderfolgender Maxima als Schätzwert für die Grundfrequenz zu bestimmen, zu mehren Maxima mit dem Schätzwert festzustellen, um die wievielte Oberwelle es sich jeweils handelt, daraus zu jedem der untersuchten Maxima eine Grundfrequenz zu bestimmen und diese zu einem Mittelwert zusammenzufassen.

5. Längenmessgerät nach einem der vorhergehenden Ansprüche, **dadurch gekennzeichnet, dass** die Steuer- und Auswerteeinheit (9) dazu eingerichtet ist, die Bandbreite des über das Mikrophon (20) erzeugten akustischen Impulses so zu wählen, dass die kleinste enthaltene Wellenlänge größer als der doppelte Durchmesser des Hohlprofils (2) ist.

6. Längemessgerät nach einem der vorhergehenden Ansprüche, **dadurch gekennzeichnet, dass** das Hohlprofil (2) auch am anderen Ende mit einer zweiten Endwand (42) versehen ist, um im Innenraum des Hohlprofils zwischen der zweiten Endwand (42) und dem inneren Schieber (6) einen zweiten akustischen Resonator (32) zu bilden, und dass die Messeinrichtung einen zweiten Lautsprecher (26) und ein zweites Mikrophon (24) im Inneren des zweiten akustischen Resonators (32) aufweist und dass die Steuer- und Auswerteeinheit (9) dazu eingerichtet ist, den zweiten Lautsprecher (26) zur Abgabe einen akustischen Impulses zu veranlassen, mit dem zweiten Mikrophon (24) die Impulsantwort des zweiten akustischen Resonators (32) aufzunehmen, daraus die Grundfrequenz des zweiten akustischen Resonators (32) zu bestimmen und aus der Grundfrequenz die Länge des zweiten Resonators (32) zu bestimmen.

7.  Längemessgerät nach Anspruch 6, **dadurch gekennzeichnet, dass** die Steuer- und Auswerteeinheit (9) dazu eingerichtet ist, mithilfe der bestimmten Längen des akustischen Resonators (30), des zweiten akustischen Resonators (32), der bekannten Länge des Hohlprofils (2) zwischen der Endwand (40) und der zweiten Endwand (42) und der bekannten axialen Länge des inneren Schiebers (6) eine Kalibrierung der Längenbestimmungen durchzuführen.

8.  Längenmessgerät nach einem der vorhergehenden Ansprüche, **dadurch gekennzeichnet, dass** die Magnetanordnung (5, 7) wenigstens einen Permanentmagneten an dem Messschieber (4) und einen Permanentmagneten an dem inneren Schieber (6) aufweist, die so angeordnet sind, dass entgegengesetzte Pole der beiden Permanentmagnete aufeinander zu weisend zueinander ausgerichtet sind.

9.  Längenmessgerät nach Anspruch 8, **dadurch gekennzeichnet, dass** jeweils vier Permanentmagnete an dem inneren Schieber (6) und an dem Messschieber (4) so zueinander angeordnet sind, dass jeweils ein Paar eines Permanentmagneten an dem Messschieber (4) und an dem inneren Schieber (6) mit entgegengesetzten Polen aufeinander zu weisend zueinander ausgerichtet sind.

10. Längenmessgerät nach einem der Ansprüche 1 bis 7, **dadurch gekennzeichnet, dass** die Magnetanordnung (5, 7) einen Permanentmagneten an einem von Messschieber und innerem Schieber aufweist und dass der andere von Messschieber und innerem Schieber ferro- oder paramagnetisches Material enthält.

11. Längenmessgerät nach einem der vorhergehenden Ansprüche, **dadurch gekennzeichnet, dass** die Messeinrichtung weiter mit einem Temperatursensor im Inneren des Hohlprofils verbunden ist und dass die Steuer- und Auswerteeinheit (9) dazu eingerichtet ist, in die Bestimmung der Länge des akustischen Resonators (30) aus dessen Grundfrequenz eine temperaturkorrigierte Schallgeschwindigkeit eingehen zu lassen.

**Claims**

1.  A length measuring device comprising a vernier caliper (4), a linear guide in the form of a hollow profile (2), on which the vernier caliper (4) is displaceably mounted externally so that it is capable of being brought into contact with an object to be measured in terms of its length, an inner slide (6), which is displaceably mounted inside the hollow profile (2), a magnet arrangement (5, 7) which couples the vernier caliper (4) and inner slide (6) magnetically, so that the inner slide (6) follows every movement of the vernier caliper (4) along the hollow profile (2), a measuring means for measuring the position of the inner slide (6) along the hollow profile (2), and a display, visible in the surrounding space of the hollow profile, of the length determined by the measuring means in accordance with the measured position of the inner slide (6), wherein the hollow profile (2) is provided with an end wall (40) on at least one end and the inner slide (6) substantially covers the cross section of the hollow profile (2) in order to form an acoustic resonator (30) in the interior of the hollow profile (2) between the end wall (40) and the inner slide (6), **characterized in that** the measuring means has a loudspeaker (22) and a microphone (20) in the interior of the acoustic resonator (30) and a control and evaluation unit (9) connected thereto, which is configured to cause the loudspeaker (22) to issue an acoustic pulse, to record the pulse response of the acoustic resonator (30) with the microphone, to determine from this the basic frequency ($f_0$) of the acoustic resonator (30) and to determine the length of the resonator (30) and therefore the position of the inner slide (6) along the hollow profile (2) from the basic frequency.

2.  The length measuring device as claimed in claim 1, **characterized in that** the control and evaluation unit (9) is configured to transform the measured pulse response of the acoustic resonator (30) into a frequency spectrum by means of a Fourier transformation and to determine the basic frequency in the frequency spectrum.

3.  The length measuring device as claimed in claim 2, **characterized in that** the control and evaluation unit (9) is configured to carry out a discrete Fourier transformation (DFT) or a fast Fourier transformation (FFT) for the Fourier transformation.

4. The length measuring device as claimed in claim 2 or 3, **characterized in that** the control and evaluation unit (9) is configured to determine the distance between two successive maxima in the frequency spectrum of the acoustic resonator (30) as an estimated value for the basic frequency, in relation to a plurality of maxima, to use the estimated value to establish the number of the harmonic involved in each case, from this to determine a basic frequency in relation to each of the maxima examined, and to combine these to form an average.

5. The length measuring device as claimed in one of the preceding claims, **characterized in that** the control and evaluation unit (9) is configured to select the bandwidth of the acoustic pulse generated via the microphone (20) such that the smallest wavelength contained is greater than twice the diameter of the hollow profile (2).

6. The length measuring device as claimed in one of the preceding claims, **characterized in that** the hollow profile (2) is also provided with a second end wall (42) on the other end in order to form a second acoustic resonator (32) in the interior of the hollow profile, between the second end wall (42) and the inner slide (6), and **in that** the measuring means has a second loudspeaker (26) and a second microphone (24) in the interior of the second acoustic resonator (32), and **in that** the control and evaluation unit (9) is configured to cause the second loudspeaker (26) to issue an acoustic pulse, to record the pulse response of the second acoustic resonator (32) with the second microphone (24), to determine the basic frequency of the second acoustic resonator (32) based on the pulse response, and to determine the length of the second resonator (32) from the basic frequency.

7. The length measuring device as claimed in claim 6, **characterized in that** the control and evaluation unit (9) is configured to carry out a calibration of the length determinations with the aid of the determined lengths of the acoustic resonator (30), of the second acoustic resonator (32), the known length of the hollow profile (2) between the end wall (40) and the second end wall (42), and the known axial length of the inner slide (6).

8. The length measuring device as claimed in one of the preceding claims, **characterized in that** the magnet arrangement (5, 7) has at least one permanent magnet on the vernier caliper (4) and a permanent magnet on the inner side (6), which are arranged in such a way that opposite poles of the two permanent magnets are aligned relative to each other so as to point toward each other.

9. The length measuring device as claimed in claim 8, **characterized in that** in each case four permanent magnets are arranged on the inner slide (6) and on the vernier caliper (4) in such a way relative to one another that in each case a pair of permanent magnets on the vernier caliper (4) and on the inner slide (6) are aligned relative to each other with opposite poles pointing toward each other.

10. The length measuring device as claimed in one of claims 1 to 7, **characterized in that** the magnet arrangement (5, 7) has a permanent magnet on one of the vernier caliper and inner slide, and **in that** the other of the vernier caliper and inner slide contains ferromagnetic or paramagnetic material.

11. The length measuring device as claimed in one of the preceding claims, **characterized in that** the measuring means is further connected to a temperature sensor in the interior of the hollow profile, and **in that** the control and evaluation unit (9) is configured to arrange for a temperature-corrected speed of sound to be incorporated into the determination of the length of the acoustic resonator (30) from its basic frequency.

**Revendications**

1. Appareil de mesure de longueur avec un pied à coulisse (4), un guidage linéaire sous la forme d'un profil creux (2), sur lequel le pied à coulisse (4) est logé de manière mobile vers l'extérieur afin de pouvoir l'amener en appui contre un objet à mesurer par rapport à sa longueur, à un coulisseau intérieur (6) qui est logé de manière mobile à l'intéueur du profil creux (2), un agencement magnétique (5, 7) qui couple magnétiquement le pied à coulisse (4) et le coulisseau intérieur (6) de sorte que le coulisseau intérieur (6) suive chaque mouvement du pied à coulisse (4) le long du profil creux (2), un dispositif de mesure pour la mesure de la position du coulisseau intérieur (6) le long du profil creux (2) et un affichage visible dans l'espace extérieur du profil creux de la longueur déterminée par le dispositif de mesure selon la position mesurée du coulisseau intérieur (6), le profil creux (2) étant pourvu, sur au moins une extrémité, d'une paroi d'extrémité (40) et le coulisseau intérieur (6) recouvrant sensiblement la section transversale du profil creux (2) afin de former dans l'espace intérieur du profil creux (2) entre la paroi d'extrémité (40) et le coulisseau intérieur (6) un résonateur acoustique (30), **caractérisé en ce que** le dispositif de mesure présente un haut-parleur (22) et un microphone (20) à l'intéueur du résonateur acoustique (30) ainsi qu'une unité de commande et d'évaluation (9) reliée à celui-ci, laquelle est aménagée afin d'amener le haut-parleur (22) à émettre une impulsion acoustique, de recevoir avec le microphone (20) la réponse d'impulsion du résonateur acoustique (30), de déterminer à partir de celle-ci la fréquence de base ($f_0$) du résonateur acoustique (30) et de déterminer à partir de la fréquence de base la longueur du résonateur (30) et ainsi la position du coulisseau intérieur (6) le long du profil creux (2).

2. Appareil de mesure de longueur selon la revendication 1, **caractérisé en ce que** l'unité de commande et d'évaluation (9) est aménagée afin de transformer la réponse d'impulsion mesurée du résonateur acoustique (30) par une transformation de Poulieur en un spectre de fréquence et de déterminer la fréquence de base dans le spectre de fréquence.

3. Appareil de mesure de longueur selon la revendication 2, **caractérisé en ce que** l'unité de commande et d'évaluation (9) est aménagée afin de réaliser pour la transformation de Fourier une transformation de Fourier discrète (DFT) ou une transformation de Fourier rapide (FFT).

4. Appareil de mesure de longueur selon la revendication 2 ou 3, **caractérisé en ce que** l'unité de commande et d'évaluation (9) est aménagée afin de déterminer dans le spectre de fréquence du résonateur acoustique (30) la distance entre deux maximum consécutifs comme valeur estimée pour la fréquence de base, de vérifier pour plusieurs des maximum avec la valeur estimée, de combien d'onde harmonique il s'agit respectivement, de déterminer à partir de là pour chacun des maximum examinés une fréquence de base et de réunir celles-ci en une valeur moyenne.

5. Appareil de mesure de longueur selon l'une quelconque des revendications précédentes, **caractérisé en ce que** l'unité de commande et d'évaluation (9) est aménagée afin de choisir la largeur de bande de l'impulsion acoustique générée par le biais du microphone (20) de sorte que la plus petite longueur d'ondes contenue soit plus grande que le double diamètre du profil creux (2).

6. Appareil de mesure de longueur selon l'une quelconque des revendications précédentes, **caractérisé en ce que** le profil creux (2) est aussi pourvu sur l'autre extrémité d'une seconde paroi d'extrémité (42) afin de former dans l'espace intérieur du profil creux entre la seconde paroi d'extrémité (42) et le coulisseau intérieur (6) un second résonateur acoustique (32) et **en ce que** le dispositif de mesure présente un second haut-parleur (26) et un second microphone (24) à l'intérieur du second résonateur acoustique (32) et **en ce que** l'unité de commande et d'évaluation (9) est aménagée afin d'amener le second haut-parleur (26) à émettre une impulsion acoustique, de recevoir avec le second microphone (24) la réponse d'impulsion du second résonateur acoustique (32), de déterminer à partir de celle-ci la fréquence de base du second résonateur acoustique (32) et de déterminer à partir de la fréquence de base la longueur du second résonateur (32).

7. Appareil de mesure de longueur selon la revendication 6, **caractérisé en ce que** l'unité de commande et d'évaluation (9) est aménagée afin de réaliser à l'aide des longueurs déterminées du résonateur acoustique (30), du second résonateur acoustique (32), de la longueur connue du profil creux (2) entre la paroi d'extrémité (40) et la seconde paroi d'extrémité (42) et de la longueur axiale connue du coulisseau intérieur (6) un calibrage des déterminations de longueur.

8. Appareil de mesure de longueur selon l'une quelconque des revendications précédentes, **caractérisé en ce que** l'agencement magnétique (5, 7) présente au moins un aimant permanent sur le pied à coulisse (4) et un aimant permanent sur le coulisseau intérieur (6) qui sont agencés de sorte que des pôles en regard des deux aimants permanents soient orientés tournés l'un vers l'autre.

9. Appareil de mesure de longueur selon la revendication 8, **caractérisé en ce que** respectivement quatre aimants permanents sont agencés les uns par rapport aux autres sur le coulisseau intérieur (6) et sur le pied à coulisse (4) de sorte que respectivement une paire d'un aimant permanent soit orientée tournés les uns vers les autres sur le pied à coulisse (4) et sur le coulisseau intérieur (6) avec des pôles en regard.

10. Appareil de mesure de longueur selon l'une quelconque des revendications 1 à 7, **caractérisé en ce que** l'agencement magnétique (5, 7) présente un aimant permanent sur un du pied à coulisse et du coulisseau intérieur et **en ce que** l'autre du pied à coulisse et du coulisseau intérieur contient un matériau ferromagnétique ou paramagnétique.

11. Appareil de mesure de longueur selon l'une quelconque des revendications précédentes, **caractérisé en ce que** le dispositif de mesure est en outre relié à un capteur de température à l'intérieur du profil creux et **en ce que** l'unité de commande et d'évaluation (9) est aménagée afin de faire entrer dans la détermination de la longueur du résonateur acoustique (30) à partir de sa fréquence de base une vitesse du son à la température corrigée.

Fig. 1

Fig. 2

Fig. 3

Fig. 4

Fig. 5

Zeit/s

Fig. 6

Fig. 7

Fig. 8

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 9817974 A1